# EUROPÄISCHE PATENTANMELDUNG

(11) **EP 0 617 002 A1**
(43) Veröffentlichungstag der Anmeldung: **28.09.1994**
(21) Anmeldenummer: 94103446.4
(22) Anmeldetag: 07.03.1994
(51) Int. Cl.: C07C 63/38, C07C 63/40, C07C 233/05, C07C 233/03, C07C 51/16, C07C 51/305

(54) **Verfahren zur Herstellung von Naphthalin-polycarbonsäuren**

(30) Priorität: 19.03.1993 DE 4308863
(71) Anmelder: BAYER AG, 51368 Leverkusen (DE)
(72) Erfinder: Roschger, Peter, Dr., D-51063 Köln (DE); Wunderlich, Klaus, Dr., D-51373 Leverkusen (DE)

(57) **Zusammenfassung**

Naphthalin-polycarbonsäuren werden dadurch erhalten, daß man Alkylnaphthaline mit einem oder mehreren Säureamiden der Formel 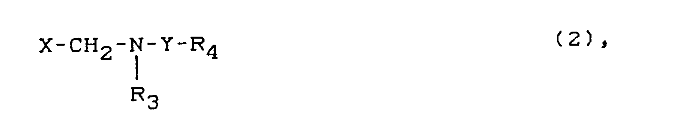
worin die Bedeutung der Substituenten der Beschreibung zu entnehmen ist, in einer Einhorn-Reaktion ein- oder mehrfach bei einer Temperatur zwischen 0 und 200 _{°} C amidomethyliert und die dabei entstandenen Zwischenprodukte oder deren Hydrolysate bzw. deren Salze zu Naphthalin-polycarbonsäuren oder deren Salzen oxidiert.

## Beschreibung

Naphthalin-polycarbonsäuren sind bedeutende Zwischenprodukte für die Herstellung von Farbstoffen und Kunststoffen.

Es sind bislang verschiedene Herstellungsverfahren für Naphthalin-polycarbonsäuren bekannt. Sie können beispielsweise durch Oxidation der entsprechenden Naphthalinpolyalkylvorläufer hergestellt werden, wie es beispielsweise in US-A-3 405 171; J. org. Chem. 30, 1453-1457 (1965) oder Bull. Chem. Soc. Jap. 45, 519-529 (1972) beschrieben ist. Hierbei sind die schwer zugänglichen isomerenreinen Alkylnaphthaline allerdings notwendig um entsprechend isomerenreine Naphthalin-polycarbonsäuren zu erhalten.

Bei der Chlormethylierung von Alkylnaphthalinen und anschließender Oxidation der Reaktionsprodukte wie sie beispielsweise aus Przem. Chem. 51, 227-230 (1972) bekannt ist, ist ein erheblicher sicherheitstechnischer Aufwand nötig, da der dabei entstehende Chlordimethylether sehr gefährlich ist.

Weiterhin ist aus US 3 137 707 ein Verfahren bekannt, nachdem aus alkylierten Benzolen durch Amidomethylierung und anschließender Oxidation Benzolcarbonsäuren erhalten werden. Die dabei zunächst gebildeten amidomethylierten Alkylbenzole fallen jedoch als Isomerengemische an.

Die vorliegende Erfindung betrifft ein Verfahren zur Herstellung von Naphthalinpolycarbansäuren, das dadurch gekennzeichnet ist, daß man Alkylnaphthaline mit einem oder mehreren Säureamiden der Formel (2) 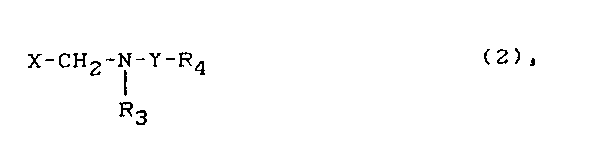
in einer Amidomethylierung nach Einhorn-Tscherniac, die im folgenden als Einhorn-Reaktion bezeichnet wird, ein- oder mehrfach bei einer Temperatur zwischen 0 und 200 °C amidomethyliert und die dabei entstandenen Zwischenprodukte oder deren Hydrolysate bzw. deren Salze zu Naphthalin-polycarbonsäuren oder deren Salze oxidiert, wobei die Substituenten in der Formel (2) folgende Bedeutung haben:
X = OH, Alkoxy, Halogen, Dialkylamino, Trialkylammonium oder cyclisches Amin, welches durch weitere Heteroatome wie beispielsweise O, N oder S, vorzugsweise O unterbrochen sein kann oder 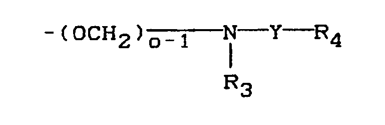
Y = CO, CS, C=N-R₅, S02,
R₃ = Wasserstoff, gegebenenfalls substituiertes C₁-C₁₀-Alkyl, -Alkenyl, -Alkinyl oder Phenyl,
R₄ = Wasserstoff, OH, gegebenenfalls substituiertes C₁-C₁₀-Alkyl, -Alkenyl, -Alkinyl, Phenyl, Amino, C, -C₄-Alkoxy, Alkoxycarbonyl oder Aminocarbonyl,
   oder
R₃ und R₄ gemeinsam den Rest eines gegebenenfalls von weiteren Heteroatomen wie beispielsweise O, S oder N, vorzugsweise N, unterbrochenen aliphatischen oder aromatischen Ring bilden,
R₅ = Wasserstoff, gegebenenfalls substituiertes C1-C6-Alkyl oder gegebenenfalls substituiertes Phenyl, wobei als Substituenten beispielsweise CI oder Methyl genannt werden können.

Eine bevorzugte Ausführungsform des zweistufigen Verfahrens zur Herstellung von Naphthalin-polycarbonsäuren, ist dadurch gekennzeichnet, daß man in der ersten Stufe Alkylnaphthaline der Formel (1) 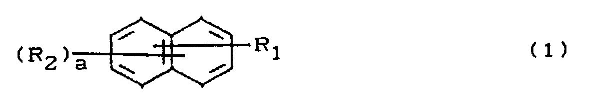
mit einem oder mehreren Säureamiden der Formel (2) 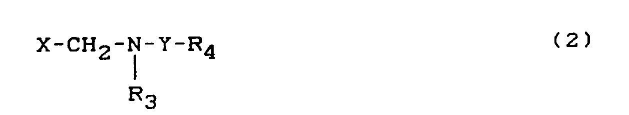
zu Verbindungen der Formel (3a) 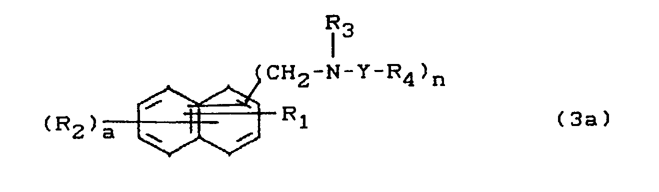
bei einer Temperatur zwischen 0 und 200 °C umsetzt und diese oder deren Hydrolysate, die den Aminen der Formel (3b) bzw. deren Salze entsprechen 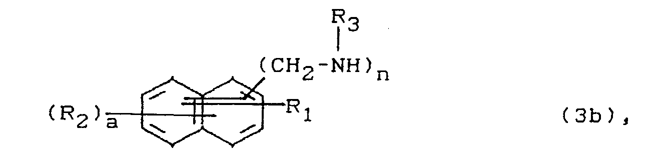
in einer zweiten Stufe zu Naphthalin-polycarbonsäuren oder deren Salze oxidiert,
worin
X, Y, R₃ und R₄ die oben genannte Bedeutung besitzen und
R₁ und R₂ = unabhängig voneinander Alkyl, vorzugsweise C₁-C₆-Alkyl bedeuten oder zusammen eine C₂-C₆-Alkyleneinheit bilden,
a = 0 oder 1 und
n = 1, 2 oder 3 bedeutet.

Die Produkte dieser Ausführungsform sind Naphthalinpolycarbonsäuren der Formel (4) 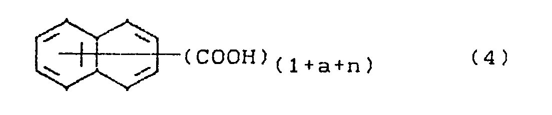
oder deren Salze.

In einer bevorzugten Ausführungsform des oben beschriebenen Verfahrens werden Säureamide der Formel (2) eingesetzt, worin Y = CO bedeutet.

In einer weiteren bevorzugten Ausführungsform dieses Verfahrens bedeuten:
Y = CO,
a = O oder 1
n = 1, 2 oder 3
R₁, R₂ = unabhängig voneinander Methyl, Ethyl, Propyl, Butyl oder zusammen eine Ethylen- oder Propylen-Einheit,
X = OH, C₁-C₄-Alkoxy, Cl, Br, Di-(G₁-C₆-alkyl)-amino, 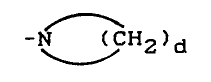 worin d = 4 bis 6, Morpholinyl, N-C₁-C₄-Alkyl-piperazinyl oder Tri-(C₁-C₆-alkyl)-ammonium mit den Gegenionen Chlorid, Bromid, lodid, Tosylat, C₁-C₆-Alkylsulfat oder Sulfat oder 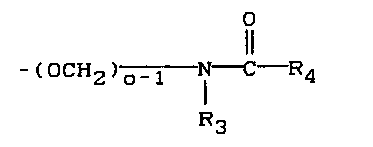
R₃ = Wasserstoff, gegebenenfalls mit Cl, OH, C1-C4-Alkoxy, Di-(C₁-C₄-alkyl)-amino, CN, C₁-C₄-Alkoxycarbonyl oder Aryl substituiertes C₁-C₁₀-Alkyl, -Alkenyl oder -Alkinyl oder gegebenenfalls durch CI oder C₁-C₄-Alkyl substituiertes Phenyl,
R₄ = Wasserstoff, gegebenenfalls durch F, Cl, Br, CN und/oder Aryl einfach oder mehrfach substituiertes C₁-C₁₀-Alkyl, -Alkenyl oder -Alkinyl, gegebenenfalls durch Cl, OH, Amino, Acylamino oder Methyl substituiertes Phenyl, Amino, Mono- oder Di-(C₁-C₄-alkyl)amino, gegebenenfalls durch Phenyl substituiertes C₁-C₄-Alkoxy, C₁-C₄-Alkoxycarbonyl, Aminocarbonyl oder OH, oder
R₃ und R₄ gemeinsam die folgenden Formeln bilden: 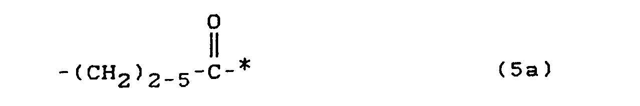 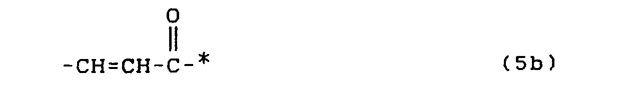 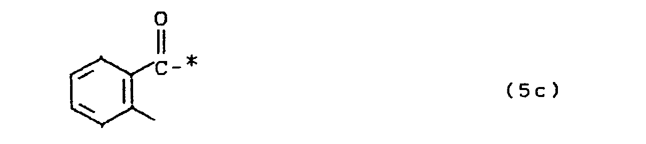 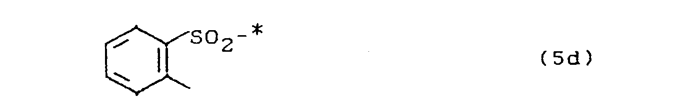 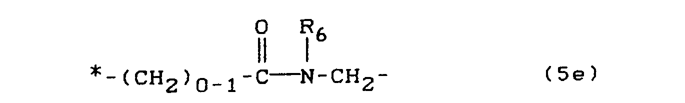 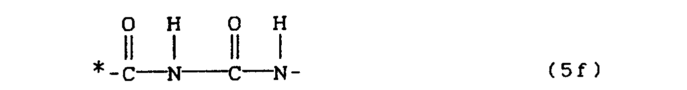 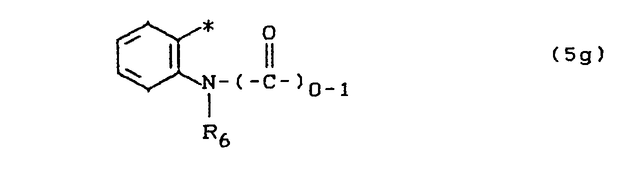
   oder
-(CH₂)₃-₅ (5h)
worin
R₆ = Wasserstoff, C₁-C₄-Alkyl, Acyl oder Aryl, mit der Vorgabe, daß die mit einem ^{*} gekennzeichneten Stellen in den Formeln (5a) bis (5g) immer mit dem N-Atom der Formel (2) bzw. (3a) verknüpft sind.

Bevorzugt werden Alkylnaphthaline aus der Gruppe: 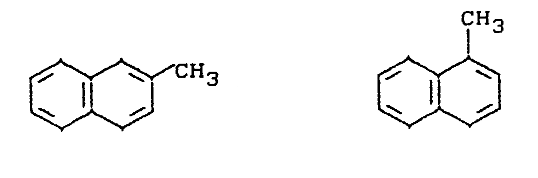 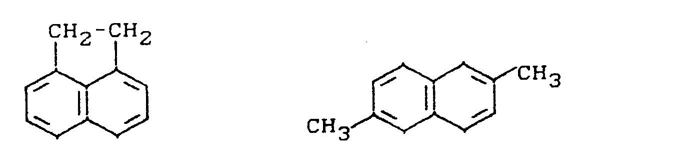 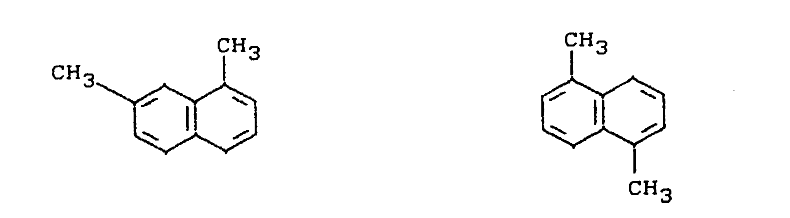 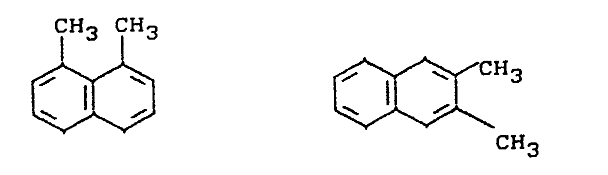 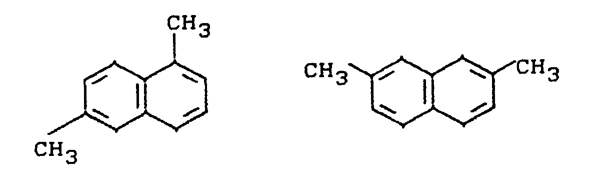
eingesetzt, insbesondere

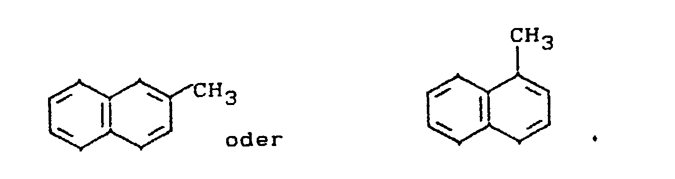

Eine ebenfalls bevorzugte Ausführungsform des erfindungsgemäßen Verfahrens verwendet Säureamide der Formel (2), worin Y = CO und X = OH bedeuten.

Basierend auf dieser Ausführungsform lassen sich weitere besonders vorteilhafte Verfahrensvarianten nennen, in denen Säureamide der Formel (2) verwendet werden, worin
R₃ = Wasserstoff
R₄ = Wasserstoff, gegebenenfalls durch CI oder Phenyl substituiertes C₁-C₄-Alkyl oder Phenyl bedeuten oder
R₃ und R₄ zusammen einen Rest der Formeln -(CH₂)₃₋₅ oder 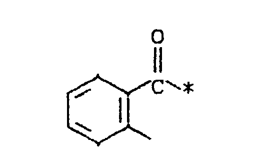
   bilden, wobei die mit ^{*} gekennzeichnete Bindung die oben genannte Bedeutung hat aber insbesondere solche Säureamide der Formel (2) verwendet werden, in denen R₃ = Wasserstoff und R₄ = Wasserstoff, Methyl oder Chlormethyl bedeutet. Besonders hervorzuheben ist dabei das Säureamid N-Methylolformamid.

Weiterhin können auch polyfunktionelle Säureamide der Formel (2) zum Einsatz kommen, worin R₃ und/oder R₄ unabhängig voneinander weitere zu einer Einhorn-Reaktion fähige Struktureinheiten der Formel 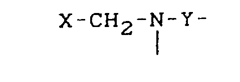
vorzugsweise 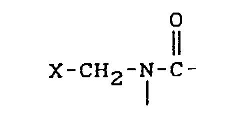
enthalten oder zusammen solche Struktureinheiten enthaltende Reste bilden.

So können beispielsweise polyfunktionelle Säureamide der Formel (2) beschrieben werden, worin 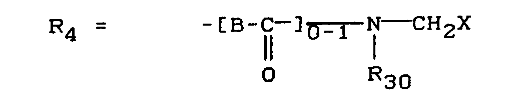
oder 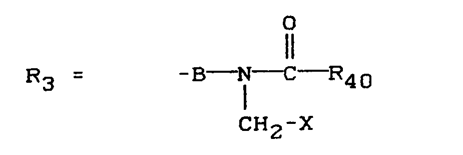
worin
R₃₀ = R₃
_{R40} = R4
und
B = direkte Bindung, gegebenenfalls eine ungesättigte Ci-C₆-Alkyleneinheit oder eine Phenylen-oder Biphenyleneinheit.

Polyfunktionelle Säureamide der Formel (2) können auch dadurch beschrieben werden, daß R₃ und R₄ zusammen beispielsweise die nachfolgenden Reste bilden: 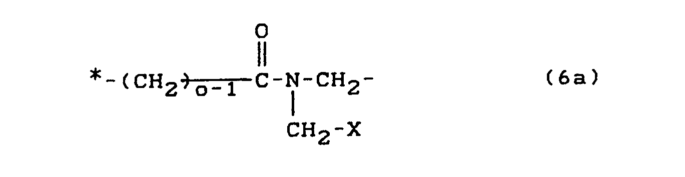 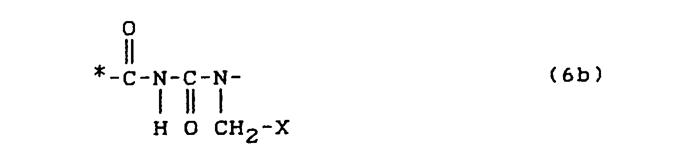 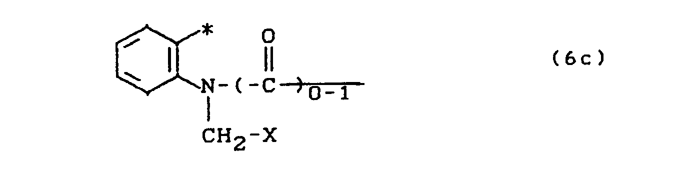 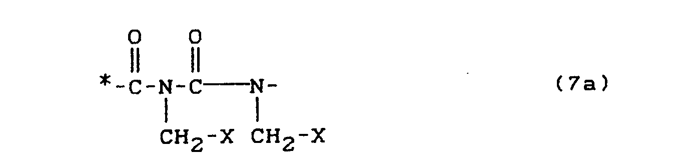
mit der Vorgabe, daß die mit einem ^{*} gekennzeichneten Stellen in den Formeln (6a) bis (6c) und (7a) immer mit dem N-Atom des Säureamids der Formel (2) verknüpft sind.

Vorzugsweise besitzen polyfunktionelle Säureamide der Formel (2) für Y = CO und X = Hydroxy. Beispielsweise wird als polyfunktionelles Säureamid folgendes eingesetzt:

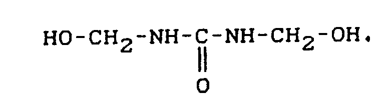

Bei den polyfunktionellen Säureamiden ist zu beachten, daß die Reste R₃ und/oder R₄ weitere zur Einhorn-Reaktion fähige Gruppen der obigen Formeln enthalten und ihre Bedeutung sich nach der Einhorn-Reaktion ändert, dadurch daß sich die Bedeutung des Substituenten X ändert. Dabei kann X die Bedeutung des eingesetzten Alkylnaphthalin der Formel (1) oder des bereits mit Säureamiden der Formel (2) ein- oder zweifach reagierten Alkylnaphthalinen der Formel (1) annehmen.

Bei der Einhorn-Reaktion erfolgt die Amidomethylierung bevorzugt in der a-Stellung des Naphthalins, ortho- oder para-ständig zum Alkylsubstituenten R₁ bzw. R₂.

Bei dem erfindungsgemäßen Verfahren können auch verschiedene Säureamide der Formel (2) in einer Mischung oder stufenweise nacheinander verwendet werden.

Die Umsetzung der Alkylnaphthaline der Formel (1) mit Säureamiden der Formel (2) zu Verbindungen der Formel (3a) in einer Einhorn-Reaktion erfolgt unter Verwendung eines Lösungsmittels und gegebenenfalls unter Mitwirkung eines Katalysators bei Temperaturen von 0 bis 200 _{°} C.

Als Lösungsmittel bei der Einhorn-Reaktion kommen all jene in Frage, die mit den Alkylnaphthalinen der Formel (1) und den Säureamiden der Formeln (2) unter den Reaktionsbedingungen keine unerwünschten Nebenreaktionen eingehen.

Beispielhaft können als geeignete Lösungsmittel genannt werden: aliphatische Alkohole, tertiäre aliphatische Amine, Ether, aliphatische und aromatische Nitroverbindungen, -Nitrile, -Sulfone, -Säureamide, Sulfoxide, Sulfonsäuren, Halogenverbindungen, Carbonsäuren und deren Anhydride, -Halogenide und -Ester, aliphatische Kohlenwasserstoffe oder einer der Reaktionskomponenten der Formeln (1) oder (2) wird im Überschuß zugegeben und dient somit als Lösungsmittel.

Im speziellen sind das z.B. Ameisensäure, Essigsäure, Propionsäure, Buttersäure und ihre Isomeren, Essigsäure-, Propionsäure- und Buttersäureanhydrid und -chlorid, Formamid, Acetamid, N-Methyl- und N,N-Dimethylformamid, N,N-Dimethylacetamid, N-Methylpyrrolidon, Pyrrolidon, Caprolactam, N-Methylcaprolactam, Tetramethylharnstoff, N,N'-Dimethylimidazolin-2-on, Dimethylsulfoxid, Acetonitril, Propionitril, Butyronitril, Benzonitril, Chlor-, Dichlor- und Trichlorbenzol, Nitrobenzol, Nitromethan, Diphenylsulfon, Sulfolan, Methansulfonsäure, Dichlormethan und -ethan, Chloroform, Tetrachlorethen, Trichlorethen, Monochlor-, Dichlor- und Trichloressigsäure und deren Anhydride und Chloride, Trifluoressigsäure, Diethylether, Tetrahydrofuran, Dioxan, Methanol, Ethanol, Propanol und Isomere, Ether von Ethylen- und Diethylenglykol, Butanole, Pentanole und Hexanole, Ethylen-, Propylen-, Diethylen- und Triethylenglykol, Triethyl-, Tri-n-propyl- und Tri-n-butyl-amin, Pyridin, Benzine in allen Siedebereichen, Essigsäureethylester und Homologe oder Hexamethylphosphorsäuretriamid.

Von besonderem Interesse sind dabei Ameisensäure, Essigsäure, Propionsäure, deren C1-C4-AlkylEster sowie Acetanhydrid.

Als Katalysatoren für die Einhorn-Reaktion kommen vorzugsweise solche mit sauren Eigenschaften in Frage wie aliphatische und aromatische Carbonsäuren und Sulfonsäuren wie deren Chloride sowie weiterhin anorganische protische und aprotische Säuren.

Im speziellen sind das z.B. Ameisensäure, Essigsäure sowie deren Mono-, Di- und Trichlorderivate, Propionsäure, Buttersäure und ihre Isomeren, Essigsäure-, Propionsäure-, Buttersäure-, Mono-, Di- und Trichloressigsäurechlorid, Methansulfonsäure, Methansulfonsäurechlorid, Benzol-, Toluol- und Naphthalinsulfonsäure und deren Säurechloride, Schwefeltrioxid, Schwefelsäure, Oleum, Chlorsulfonsäure, Thionylchlorid, Sulfurylchlorid, Phosphorsäure, Polyphosphorsäure, Phosphorpentoxid, Phosphortri- und -pentachlorid, Phosphortribromid, Phosphoroxytrichlorid und -tribromid, Fluor-, Chlor-, Brom- und lodwasserstoff, Aluminium-, Eisen-III-fluorid, -chlorid und -bromid, Antimon-III- und -V-fluorid, saures Aluminiumoxid, Zinntetrachlorid, Bortrifluorid und -chlorid, Siliciumtetrachlorid, Methylchlor-silane, Perchlorsäure, Arsen-III- und -V-fluorid, Arsen-III-chlorid, Antimon-III-chlorid, Zinkchlorid, Titan-IV-chlorid, usw..

Von besonderen Interesse sind dabei vor allem Schwefelsäure, Oleum, Phosphorsäure oder Polyphosphorsäure.

Als bevorzugte Lösungsmittel-Katalysator-Kombination ist beispielsweise eine Mischung aus Essigsäure (95 bis 100 %ig) und Schwefelsäure (90 bis 100 %ig) zu nennen, die in einem Volumenverhältnis von 90:10 bis 50:50 variiert werden kann.

Eine ebenso bevorzugte Kombination ist eine Mischung aus Propionsäure (95 bis 100 %ig) oder Essigsäureethylester mit Schwefelsäure (90 bis 100 %ig) in dem oben angegebenen Bereich der Mischungsverhältnisse.

Die Einhorn-Reaktion wird im allgemeinen bei Temperaturen von 0 bis 200 _{°} C, vorzugsweise bei 0 bis 120°C, besonders bevorzugt bei 15 bis 70 _{°} C durchgeführt.

Die Reaktion wird im allgemeinen unter Normaldruck durchgeführt, kann aber bei Verwendung von niedersiedenden Lösungsmitteln gegebenenfalls auch bei erhöhten Drucken beispielsweise im Autoklaven durchgeführt werden.

Die verwendeten Säureamide der Formeln (2) werden im allgemeinen in einem bis zu 30 %igen molaren Überschuß bezogen auf die gewünschte Größe von n eingesetzt, sofern nicht ein Reaktionspartner gleichzeitig als Lösungsmittel dient.

In einer ganz besonderen Ausführungsform des Verfahrens werden Säureamide der Formel (2) mit X = Hydroxy eingesetzt, die zusammen mit dem Alkylnaphthalinen der Formel (1) bei einer Temperatur von 0 bis 80 ° C in einem Lösungsmittel bestehend aus H2S04 und niederen Carbonsäuren oder deren Ester bzw. Anhydriden oder inerten Verdünnungsmittel umgesetzt werden, und anschließend isoliert werden.

Die Aufarbeitung der Einhorn-Reaktion erfolgt für X = OH im allgemeinen dadurch, daß man aus der Reaktionslösung die Verbindung der Formel (3a) mit Wasser fällt. Die Reaktionslösung kann dazu vorher durch teilweise oder vollständige Entfernung des Lösungsmittels aufkonzentriert werden. Verbindungen der Formel (3a) die in der Reaktionslösung schwer löslich sind, können direkt abfiltriert werden, ohne daß man Wasser zusetzt.

Ein Sonderfall des Verfahrens ist der, daß man die Reaktionslösung nach der Einhorn-Reaktion, vorzugsweise nach vorherigem Entfernen des Lösungsmittels beispielsweise durch Destillation bei vermindertem Druck, mit Wasser versetzt und diese Mischung bei erhöhter Temperatur, insbesondere bei 50 bis 150 _{°} C, gegebenenfalls unter Druck erhitzt, wobei Hydrolysate der Formel (3b) oder deren Salze erhalten werden.

Diese Hydrolysate der Formel (3b) sind freie, nicht acylierte Amine bzw. deren Salze und können der nachfolgenden Oxidation in gleicher Weise zugeführt werden wie die Verbindungen der Formel (3a).

In der 2. Stufe des Verfahrens, der Oxidation, werden die aus der vorhergehenden Einhorn-Reaktion erhaltenen Verbindungen der Formel (3a) oder deren Hydrolysate der Formel (3b) bzw. deren Salze zu Naphthalin-polycarbonsäuren der Formel (4) mit einem Oxidationsmittel gegebenenfalls in Gegenwart eines Katalysators in einem Lösungsmittel oxidiert.

Als Oxidationsmittel kommen beispielsweise in Betracht:
Sauerstoff, auch in Form von Luft, Chrom-VI-Verbindungen, Mangan-IV- und -VII-Verbindungen, anorganische und organische Peroxide, Stickstoff-Sauerstoff-Verbindungen, Halogensauerstoffverbindungen, Halogene, Selen-IV-Verbindungen.

Speziell genannt seien Sauerstoff, Luft, Natrium-, Kalium- und Ammoniumchromat und -dichromat, Chromsäure, Braunstein, Kaliumpermanganat, Wasserstoffperoxid, Peressigsäure, Natriumperoxid, Natrium-, Kalium- und Ammoniumperoxodisulfat, Natriumperborat, Natriumhypochlorit, Natriumchlorit, Natrium- und Kaliumchlorat, Salpetersäure, Stickstoffdioxid, Distickstofftrioxid, -tetroxid und -pentoxid, Chlor, Brom, Fluor, Selendioxid etc.

Von besonderem Interesse sind Alkalichromate und -bichromate, insbesondere Natriumbichromat.

In einzelnen Fällen ist die Verwendung eines Oxidationskatalysators vorteilhaft, wofür vor allem Verbindungen und Salze von Übergangsmetallen wie z.B. Vanadium, Wolfram, Molybdän, Kobalt, Nickel, Eisen, Mangan, Osmium, Palladium, Platin etc, in Frage kommen.

Als Lösungsmittel für die Oxidation können all jene herangezogen werden, die sich gegenüber dem Oxidationsmittel als inert erweisen.

Bevorzugt sind Lösungsmittel wie Wasser, aliphatische Carbonsäuren, wäßrige Mineralsäuren oder Alkali-/Erdalkalilaugen, Halogenaromaten, Halogenaliphaten usw.. In wäßrigen Phasen kann der Zusatz von inerten Emulgatoren vorteilhaft sein. Die Oxidation kann in Abhängigkeit vom Oxidationsmittel und Lösungsmittel im allgemeinen bei Temperaturen zwischen 0 und 350 _{°} C, vorzugsweise zwischen 20 und 300 _{°} C, besonders bevorzugt zwischen 50 und 250 _{°} C durchgeführt werden.

Gegebenenfalls kann es vorteilhaft sein, die Oxidation unter erhöhtem Druck durchzuführen, vorzugsweise bei einem Druck von 1 bis 30 bar.

Die Naphthalinpolycarbonsäuren fallen in hoher Reinheit an, Eine weitergehende Reinigung kann nach für aromaische Carbonsäuren übliche Methoden wie z.B. Lösen in Laugen und Ausfällen mit Säuren, Umkristallisation, Lösungsmittelwäschen, Derivatisierung z.B. Veresterung und anschließender Verseifung oder chromatografische Methoden usw. erfolgen.

Vorteilhaft an diesem Verfahren ist, daß Mono- und Dialkylnaphthaline der Formel (1) in ausreichendem Maße verfügbar sind und Säureamide der Formeln (2) gut zugänglich sind, vgl. Liebigs Ann. Chem. 343. 207 (1905); Ibid. 361, 113 (1908); Ibid. 583, 37 (1953); US 3.073.843; DE 3.700.457 (1988).

Gegenüber einem bestehenden Verfahren der direkten Oxidation von Polyalkyl-naphthalinen zu den entsprechenden Carbonsäuren (US-A-3.405.171, J. org. Chem. 30, 1453-1457 (1965), Bull. Chem. Soc. Jap. 45, 519-529 (1972) oder Przem. Chem. 51, 227-230 (1972)), zeigt das neue Verfahren den Vorteil, daß man Alkylnaphthaline mit einer niedrigeren Anzahl von Alkylgruppen einsetzt, welche in größeren Mengen und zu geringeren Preisen am Weltmarkt erstanden werden können, als höher alkylierte Naphthaline. Die erste Stufe des neuen Verfahrens zeigt zusätzlich den Vorteil, daß isomerenreine Produkte erhalten werden, im Gegensatz zur direkten Alkylierung von Naphthalin und seinen Alkylderivaten, die immer komplexe Isomerengemische liefern (vgl. z.B. JP 04139134 (1992), JP 63014738 (1988), JP 63014739 (1988); siehe auch Chem. Ber. 84, 826 bis 831 (1951)).

Gegenstand der Erfindung sind weiterhin neue Verbindungen der Formel (3a), 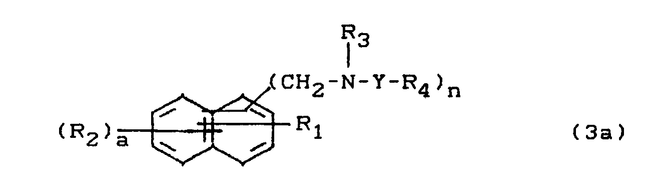
worin
Ri, R₂, R₃, R₄, a, n und Y die obengenannten Bedeutungen haben, mit Ausnahme der folgenden Verbindungen 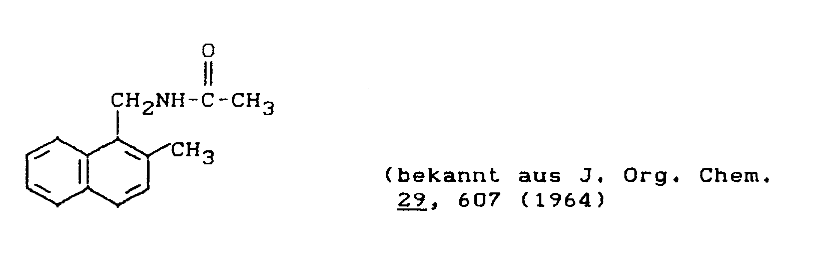 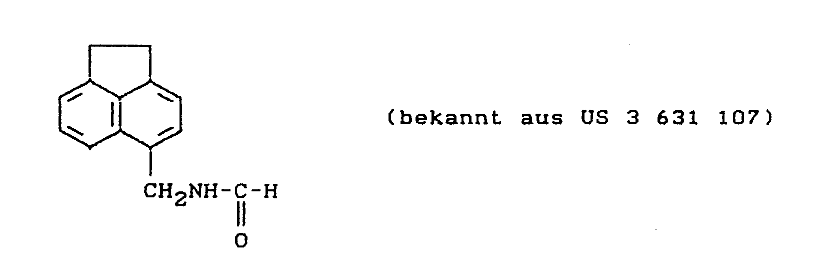
   und

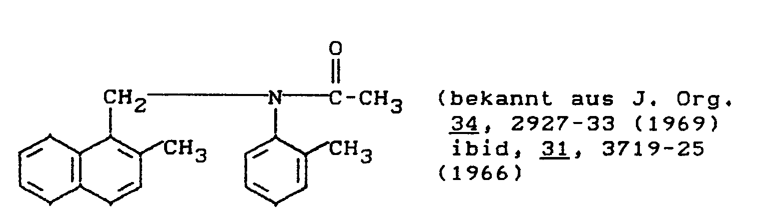

Neben den bevorzugten Ausführungsformen für Verbindungen der Formel (3a), die sich aus den oben genannten Vorzugsbereichen der Substituenten R₁, R₂, R₃, R₄, a, n und Y ergeben - exklusive der genannten Ausnahmen - sind besonders Verbindungen der Formel (3a) bevorzugt, worin
R₁ und R₂ Methyl oder Ethyl bedeuten oder zusammen einen Ethylen-Rest bilden,
a für 0 oder 1 steht,
R₃ Wasserstoff,
R₄ Wasserstoff, gegebenenfalls durch Chlor oder Phenyl substituiertes C1-C4-Alkyl oder Phenyl bedeutet oder
R₃ und R₄ zusammen Reste der Formel -(CH₂)_{3̅-̅5̅} oder 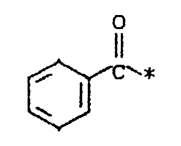 bilden, wobei ^{*} die oben genannte Bedeutung hat,
Y CO bedeutet und
n gleich 1 ist, wenn a gleich 0 ist, bzw.
n gleich 1 oder 2 ist, wenn a gleich 1 ist.

Beispielsweise können solche Verbindungen wie die folgenden genannt werden:

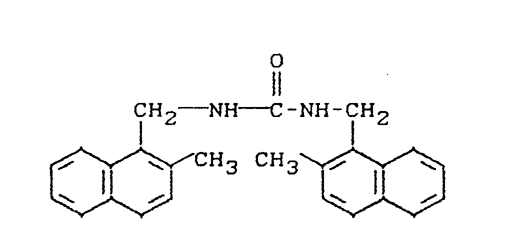

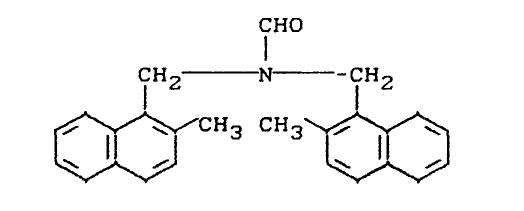

Ganz besonders bevorzugte Verbindungen der Formel (3a) sind solche, worin
Y = für CO,
a = für O,
R₁ = für Methyl,
R₃ = für Wasserstoff,
R₄ = für Wasserstoff, Methyl oder Chlormethyl steht und n gleich 1 bedeutet.

Gegenstand der Erfindung sind weiterhin neue Verbindungen der Formel (3b) oder deren Salze 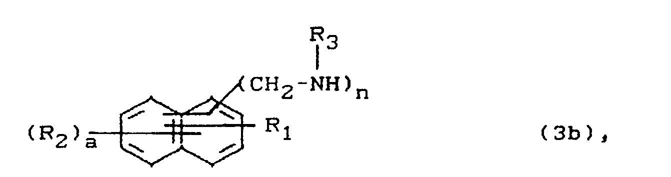
worin
R₁, R₂ , R₃, a und n die oben genanntene Bedeutung haben mit Ausnahme der folgenden Verbindungen. 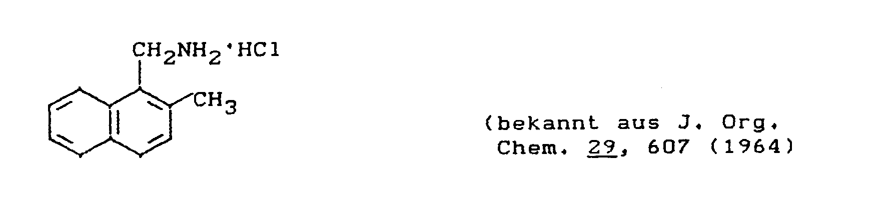 und

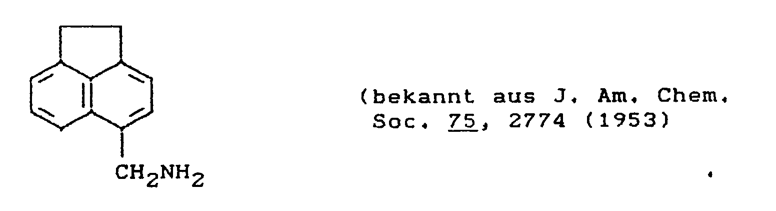

Neben den bevorzugten Ausführungsformen für Verbindungen der Formel (3b), die sich aus den oben genannten Vorzugsbereichen der Substituenten R₁, R₂, R₃, a und n ergeben, - exkluxive der genannten Ausnahmen - sind besonders Verbindungen der Formel (3b) sowie die mineralsauren Salze dieser Hydrolysate bevorzugt, worin
R₁ und R₂ Methyl oder Ethyl bedeuten oder zusammen einen Ethylen-Rest bilden
a für 0 oder 1 steht,
R₃ Wasserstoff bedeutet,
n gleich 1 ist, wenn a gleich 0 ist bzw.
n gleich 1 oder 2 ist, wenn a gleich 1 ist.

Beispielsweise ist dies eine Verbindung der Formel

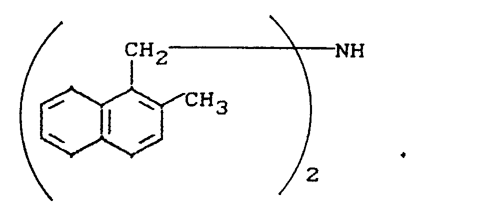

Besonders bevorzugt sind auch jene Verbindungen der Formel (3b) in Form ihrer schwefel- oder phosphorsauren Salze, worin
a gleich 0 ist
R₁ gleich Methyl,
R₃ Wasserstoff bedeuten und
n gleich 1 ist.

### Beispiel 1

### N-Methylolformamid

Eine Suspension von 44 g Paraformaldehyd in 80 ml Formamid wird tropfenweise mit 0,8 ml 40 %iger Natronlauge versetzt. Dabei geht der Paraformaldehyd nach und nach unter Selbsterwärmung (bei kleineren Ansätzen externe Erwärmung notwendig) des Ansatzes auf 40 bis 50 °C in Lösung. Es wird noch 15 Minuten nachgerührt und die erhaltene Lösung direkt für die nachfolgenden Umsetzungen verwendet.

### Stufe 1

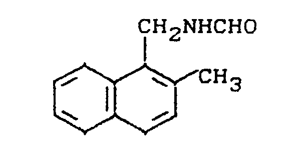

Zu einer Mischung aus 170,4 g 2-Methylnaphthalin, 640 ml Eisessig und 160 ml 100 %ige Schwefelsäure wird bei 40 bis 45 _{°} C die oben dargestellte N-Methylolformamid-Lösung in 20 Minuten zugetropft. Nach 6 Stunden gießt man die Losung auf 4 I Eiswasser und saugt den Niederschlag nach 12 Stunden ab und wäscht mit Wasser neutral. Trockenausbeute 200 bis 220 g.

### Stufe 2

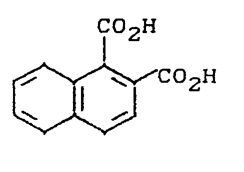

Der feuchte Preßkuchen aus Stufe 1 wird mit 746 g Natriumbichromatdihydrat und 1,3 I Wasser im Autoklaven 48 Stunden auf 200 bis 210°C erhitzt. Nach dem Abkühlen und Entspannen saugt man vom Chromoxid ab und säuert das Filtrat mit Schwefelsäure auf pH 1 an. Der ausgefallene Niederschlag wird abgesaugt und mit Wasser bis zum farblosen Ablauf gewaschen.

Ausbeute 145 g.

Für die Oxidation kann auch das getrocknete Produkt aus Stufe 1 eingesetzt werden, ohne das Ergebnis zu beeinträchtigen.

### Beispiel 2

Ersetzt man die in Beispiel 1 angegebene Volumenmenge an 100 %iger Schwefelsäure durch 96 %ige Schwefelsäure, so erhält man 200 bis 210 g des in Beispiel 1, Stufe 1 beschriebenen Zwischenproduktes. Die Oxidatin analog zu Beispiel 1, Stufe 2 liefert 142 g Naphthalin-1,2-dicarbonsäure.

### Beispiel 3

Ersetzt man die in Beispiel 1 angegebene Volumenmenge an Essigsäure durch Propionsäure, so erhält man 195 g der in Beispiel 1, Stufe 1 beschriebenen Verbindung. Die entsprechende Oxidation liefert 133 g Naphthalin-1,2-dicarbonsäure.

### Beispiel 4

Ersetzt man die Eisessig-Menge in Beispiel 1 durch 560 ml Eisessig und 80 ml Acetanhydrid, so erhält man ca. 200 g der in Beispiel 1, Stufe 1 beschriebenen Verbindung.

Die analoge Oxidation liefert 128 g Naphthalin-1,2-dicarbonsäure.

### Beispiel 5

Ersetzt man die Lösungsmittelmischung Eisessig-Schwefelsäure in Beispiel 1, Stufe 1 durch 800 ml Ameisensäure, erhitzt 6 Stunden unter Rückfluß und arbeitet wie in Beispiel 1, Stufe 1 beschrieben auf, so erhält man 190 g des dort angegebenen Produktes. Oxidation analog zu Beispiel 1, Stufe 2 liefert 105 g Naphthalin-1,2-dicarbonsäure.

### Beispiel 6

Ersetzt man die Schwefelsäure-Menge in Beispiel 1, Stufe 1 durch 300 g Polyphosphorsäure, so werden 199 g des dort beschriebenen Produktes erhalten.

Oxidation nach Beispiel 1, Stufe 2 führt zu 118 g Naphthalin-1,2-dicarbonsäure.

### Beispiel 7

### Stufe 1

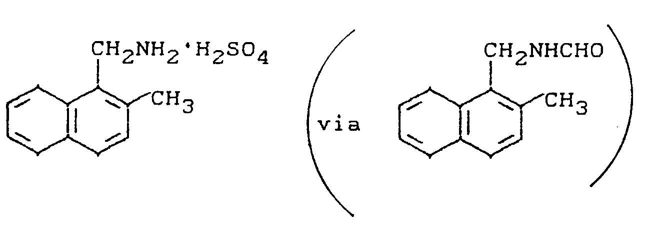

Eine Mischung aus 80 ml Essigsäureethylester, 20 ml 100 %iger Schwefelsäure und 21,3 g 2-Methylnaphthalin wird bei unter 30°C mit N-Methylolformamid-Lösung (aus 10 ml Formamid, 5,75 g Paraformaldehyd und 0,1 ml einer 40 Gew.-%igen NaOH-Lösung) versetzt. Nach 4 Std Reaktion bei 40 °C wird der Essigester im Vakuum bei unter 35°C Innentemperatur abdestilliert. Der Rückstand wird dann nach und nach mit 100 ml Wasser vorsichtig versetzt (bei der dabei entstehenden Fällung handelt es sich um 1-Formylaminomethyl-2-methylnaphthalin, welches abgesaugt und anschließend der Oxidation analog zu Beispiel 1, Stufe 2 unterworfen werden kann). Die Suspension wird unter Rühren 2 Stunden zum Sieden erhitzt. Nach dem Erkalten kühlt man auf 0°C ab und saugt das Amin-Sulfat nach einigen Stunden scharf ab. Ausbeute: 29,0 g

### Stufe 2

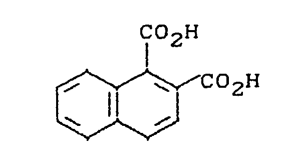

26,9 g des Produktes aus Stufe 1 (Schwefelsaures Salz des 1-Aminomethyl-2-methyl-naphthalin), 150 ml Wasser und 74,6 g Natriumbichromatdihydrat werden 48 Stunden auf 200 bis 210°C erhitzt. Aufarbeitung analog zu Beispiel 1, Stufe 2 liefert 17,8 g 1,2-Naphthalindicarbonsäure.

### Beispiel 8

### Stufe 1

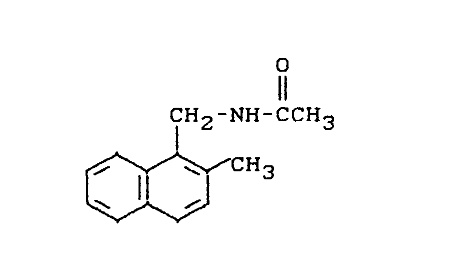

In eine Mischung aus 21,3 g 2-Methylnaphthalin, 80 ml Eisessig und 20 ml konzentrierter Schwefelsäure werden bei 40 °C in 10 Minuten 13,4 g N-Methylolacetamid eingetragen.

Nach 6 Stunden wird der Niederschlag abgesaugt und gut mit H₂0 gewaschen.

Ausbeute 21,9 g.

### Stufe 2

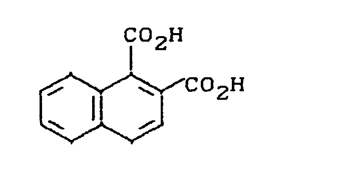

Eine Mischung aus 10,7 g des Produktes aus Stufe 1, 37,7 g Natriumbichromatdihydrat und 75 ml Wasser wird im Autoklaven 24 Stunden auf 200 bis 210_{°}C erhitzt. Aufarbeitung analog zu Beispiel 1, Stufe 2 liefert 7,7 g Naphthalin-1,2-dicarbonsäure.

### Beispiel 9

### Stufe 1

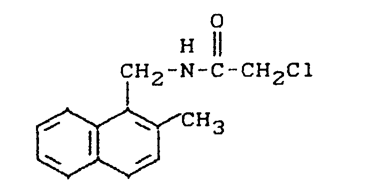

Ersetzt man in Beispiel 8 das N-Methylolacetamid durch 18,7 g N-Methylol-chloracetamid und arbeitet weiter wie dort beschrieben, so erhält man 25,1 g des obigen Produktes.

### Stufe 2

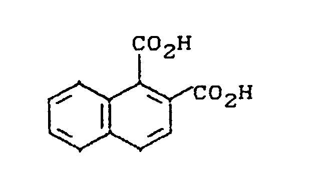

Die zu Beispiel 8, Stufe 2 analoge Oxidation von 12,4 g des Produktes aus Stufe 1 liefert 7,9 g Naphthalin-1,2-dicarbonsäure.

### Beispiel 10

### Stufe 1

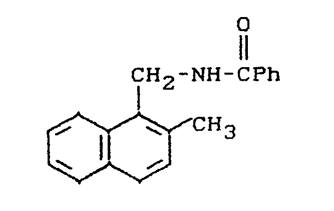

Eine Mischung von 21,3 g 2-Methylnaphthalin, 80 ml Eisessig und 20 ml 100 %ige Schwefelsäure wird während 45 Minuten mit 24,9 g N-Methylolbenzamid versetzt. Nach 6 Stunden bei 40 °C trägt man auf 500 ml Eiswasser aus und saugt nach einigen Stunden ab. Ausbeute 37,0 g.

### Stufe 2

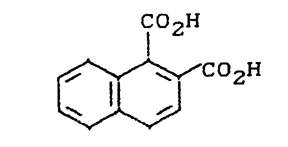

27,5 g des Produktes aus Stufe 1, 150 ml Wasser und 74,6 g Natriumbichromatdihydrat werden in einem Autoklaven 48 Stunden auf 200 bis 210_{°}C erhitzt. Nach dem Abkühlen und Entspannen wird vom Chromoxid abgesaugt und das Filtrat stark angesäuert. Der Niederschlag wird abgesaugt und mit Wasser gewaschen. Zur Entfernung anhaftender Benzoesäure kristallisiert man aus viel Wasser um.

Ausbeute 12,9 g.

### Beispiel 11

### Stufe 1

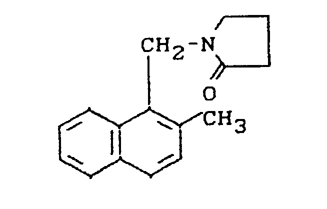

Eine Mischung aus 21,3 g 2-Methylnaphthalin, 80 ml Eisessig, 20 ml 100 %iger Schwefelsäure und 19,0 g N-Methylolpyrrolidon wird 1,5 Stunden bei 40 °C gehalten und dann auf 500 ml Eis ausgetragen. Dabei fällt das Produkt als Öl an, welches direkt zur Oxidation verwendet werden kann.

Ein kristallines Produkt erhält man wie folgt:

Das Öl wird in Methylenchlorid aufgenommen, mit Natriumbicarbonat-Lösung neutral gewaschen und über Natriumsulfat getrocknet. Die Lösung wird anschließend im Vakuum vom Lösungsmittel befreit und der harzige Rückstand mit t-Butylmethylether digeriert. Dabei tritt Kristallisation ein. Das Produkt wird abgesaugt und mit wenig t-Butylmethylether und Ligroin gewaschen.

Ausbeute 25,4 g.

### Stufe 2

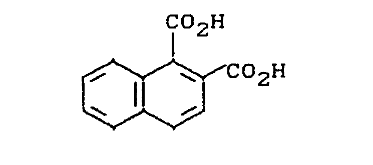

Die Oxidation des öligen Rohproduktes mit 74,6 g Natriumbichromatdihydrat in 150 ml Wasser im Autoklaven (48 Stunden, 200 bis 210_{°}C) und Aufarbeitung auf übliche Weise liefert 16,5 g Naphthalin-1,2-dicarbonsäure.

### Beispiel 12

### Stufe 1

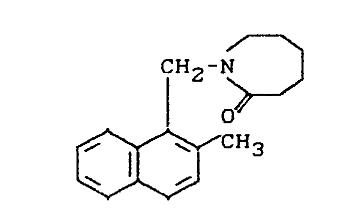

Die Darstellung erfolgt auf gleiche Weise, wie es in Beispiel 11 beschrieben wird, unter Verwendung von 25,9 g N-Methylolcaprolactam anstelle von N-Methylolpyrrolidon.

Ausbeute (kristallines Produkt) 27,1 g.

### Stufe 2

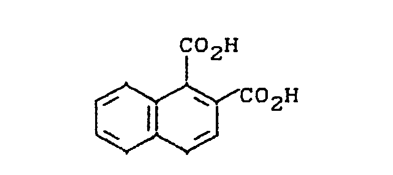

Die Oxidation des öligen Rohproduktes der Stufe 1 analog zu Beispiel 11 liefert 16,0 g Naphthalin-1,2-dicarbonsäure.

### Beispiel 13

### Stufe 1

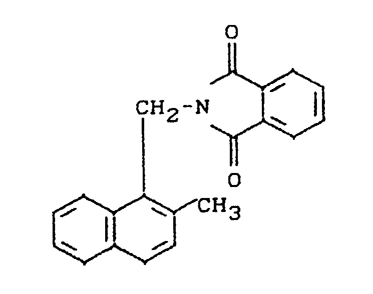

29,2 g N-Methylolphthalimid werden während 30 Minuten bei ca. 40 °C in eine Mischung aus 21,3 g 2-Methylnaphthalin, 80 ml Eisessig und 20 ml konzentrierter Schwefelsäure eingetragen. Nach 5 Stunden trägt man auf 500 ml Eiswasser aus, saugt den Niederschlag ab und trocknet im Vakuum bei 80 °C. Das Rohprodukt wird in Eisessig gelöst, filtriert, das Produkt mit Eiswasser gefällt und abgesaugt; Ausbeute 35,8 g^{.}

### Stufe 2

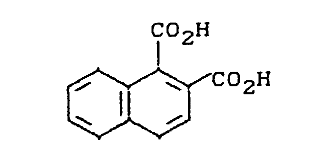

Oxidation entsprechend den vorstehenden Methoden liefert Naphthalin-1,2-dicarbonsäure, die wechselnde Mengen Phthalsäure enthält.

### Beispiel 14

### Stufe 1

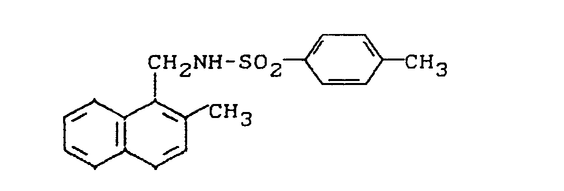

Eine Mischung von 10,6 g 2-Methylnaphthalin, 40 ml Eisessig, 10 ml 100 %ige Schwefelsäure und 16,6 g N-Methyloltosylamid wird 5 Stunden auf 40 _{°} C erhitzt. Man filtriert vom ausgefallenen produkt und wäscht es auf Eisessig und Wasser nach; Ausbeute 14,5 g.

### Stufe 2

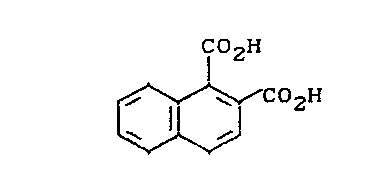

Die Oxidation des Produktes analog zu den vorstehenden Beispielen liefert 2,5 bis 4,8 g Naphthalin-1,2-dicarbonsäure.

### Beispiel 15

### Stufe 1

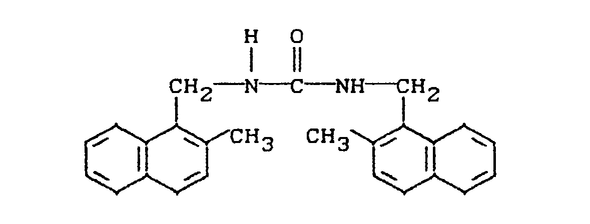

Eine Mischung aus 21,3 g 2-Methylnaphthalin, 80 ml Eisessig, 20 ml 100%ige Schwefelsäure und 9,0 g Dimethylolharnstoff wird 6 Stunden auf 40 bis 50°C erhitzt. Anschließend filtriert man heiß von entstandenen Nebenprodukten ab und fällt das Produkt aus der Eisessiglösung mit 500 ml Eiswasser. Man saugt das Produkt ab und wäscht es mit Wasser. Ausbeute: 12,8 g.

### Stufe 2

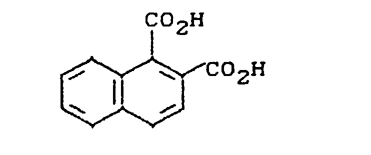

Ein Ansatz von 12,0 g des obigen Produktes, 50,0 g Natriumbichromatdihydrat und 120 ml Wasser wird 48 Stunden auf 200 bis 210°C erhitzt und danach in üblicher Weise durch Absaugen vom Chromoxid und Ansäuern des Filtrates aufgearbeitet. Ausbeute 6,3 g.

### Beispiel 16

### N,N-Dimethylolformamid

22,5 g Formamid, 31,0 g Paraformaldehyd und 0,3 g Magnesiumoxid werden 20 Minuten auf 100°C erhitzt. Nach dem Erkalten kann das ölige Produkt direkt eingesetzt werden.

### Stufe 1

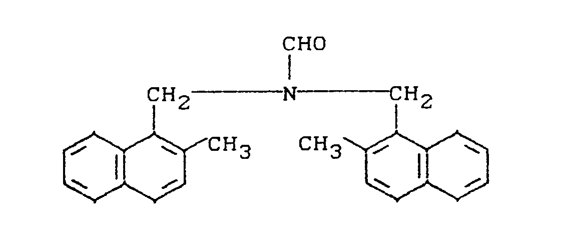

Eine Mischung aus 21,3 g 2-Methylnaphthalin, 80 ml Eisessig, 20 ml 100%iger Schwefelsäure und 7,9 g N,N-Dimethylformamid-Lösung (obiges Rohprodukt) werden 2 Stunden auf 40 bis 50°C erhitzt und dann auf Wasser ausgetragen. Das zäh-harzige Rohprodukt wird in Aceton gelöst und mit H₂0 langsam wieder ausgefällt. Ausbeute 24,0 g.

### Stufe 2

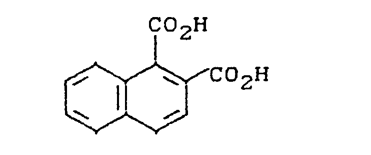

17,6 g des obigen Produktes werden entsprechend Beispiel 11, Stufe 2 oxidiert und aufgearbeitet. Ausbeute 9,2 g.

### Beispiel 17

### Stufe 1

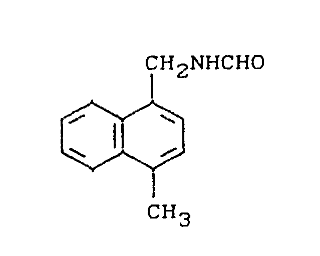

Zu einer Mischung von 21,3 g 1-Methylnaphthalin, 80 ml Essigsäure und 20 ml konzentrierter Schwefelsäure wird in 15 Minuten bei 40 _{°} C eine Methylolformamid-Lösung (hergestellt aus 11 ml Formamid, 6,3 g Paraformaldehyd und 0,1 ml 40 %iger Natronlauge) getropft. Nach 6 Stunden bei 40°C trägt man die Mischung auf Eis (500 g) aus und saugt den Niederschlag ab. Ausbeute 24,7 g.

### Stufe 2

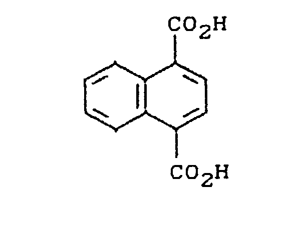

10 g des Produktes aus Stufe 1 werden mit 37,3 g Natriumdichromatdihydrat und 75 ml Wasser im Autoklaven 24 Stunden auf 200 bis 210°C erhitzt. Nach dem Abfiltrieren vom Chromoxid säuert man das Filtrat mit 60 ml 15 %iger Schwefelsäure an, saugt den Niederschlag ab und wäscht mit Wasser bis zum farblosen Ablauf. Ausbeute 8,6 g.

### Beispiel 18

### Stufe 1

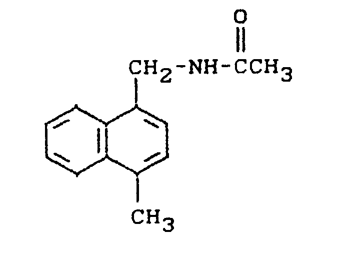

Eine Mischung aus 21,3 g 1-Methylnaphthalin, 80 ml Eisessig, 20 ml konzentrierte Schwefelsäure und 17,5 g N-Methylolacetamid wird 5 Stunden bei 40 bis 50 °C gehalten und danach auf Eis (500 g) ausgetragen. Der Niederschlag wird abgesaugt und mit Wasser gewaschen. Ausbeute 28,8 g.

### Stufe 2

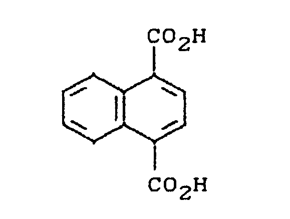

Oxidation von 10,7 g des Produktes der Stufe 1 analog zu Beispiel 17, Stufe 2 liefert 9,0 g Naphthalin-1,4-dicarbonsäure.

### Beispiel 19

### Stufe 1

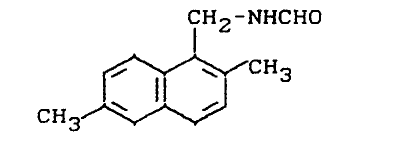

Zu einer Mischung von 23,4 g 2,6-Dimethylnaphthalin, 80 ml Eisessig und 20 ml konzentrierter Schwefelsäure wird bei 40°C während 15 Minuten eine N-Methylolformamid-Lösung (aus 10 ml Formamid, 5,75 g Paraformaldehyd und 0,1 ml 40 %iger Natronlauge) zugetropft. Man hält 6 Stunden bei 40 _{°} C und gießt den Ansatz danach auf 500 ml Eiswasser. Man dekantiert vom öligen Produkt, löst es anschließend in 400 ml Methanol und filtriert vom unlöslichen Rückstand (Bis-Produkt). Zum methanolischen Filtrat tropft man Wasser, wobei es anfang zu öligen Abscheidungen kommt, die man wiederholt abtrennt, bis es zur kristallinen Fällung des Produktes kommt, die man durch weitere Wasserzugabe vervollständigt. Das Produkt wird abgesaugt und mit Wasser gewaschen; Ausbeute 7,7 g.

### Stufe 2

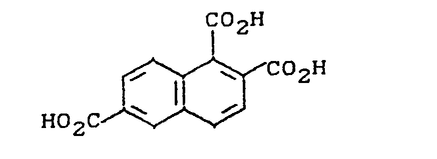

Eine Mischung aus 7,1 g des obigen Produktes, 37,3 g Natriumbichromatdihydrat und 75 ml Wasser wird im Autoklaven 24 Stunden auf 200 bis 210°C erhitzt. Anschließend reduziert man den Chromatüberschuß mit Natriumbisulfit-Lösung, versetzt mit 3 g Aktivkohle und erhitzt auf 75 °C. Nach dem Klären säuert man das Filtrat mit 50 ml konzentrierter Salzsäure an, saugt den Niederschlag ab und wäscht mit Wasser nach. Ausbeute 3,0 g.

### Beispiel 20

### Stufe 1

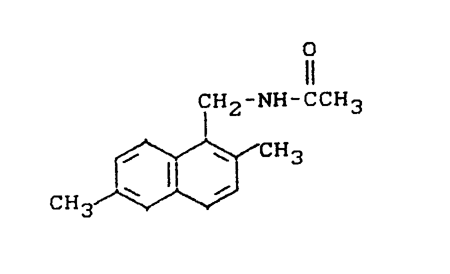

Eine Mischung aus 23,4 g 2,6-Dimethylnaphthalin, 80 ml Eisessig, 20 ml konzentrierter Schwefelsäure und 13,4 g N-Methylolacetamid wird 6 Stunden bei 40 °C gehalten und anschließend vom Niederschlag abfiltriert (Bis-Produkt). Das Filtrat wird auf 500 ml Eiswasser gegossen und der Niederschlag nach einigen Stunden abgesaugt. Ausbeute 24,4 g.

### Stufe 2

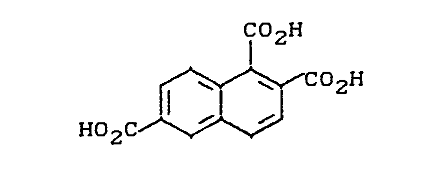

Oxidation von 7,6 g des Produktes aus Stufe 1 nach dem Verfahren in Beispiel 19, Stufe 2 liefert 4,1 g Naphthalin-1,2,6-tricarbonsäure.

### Beispiel 21

### Stufe 1

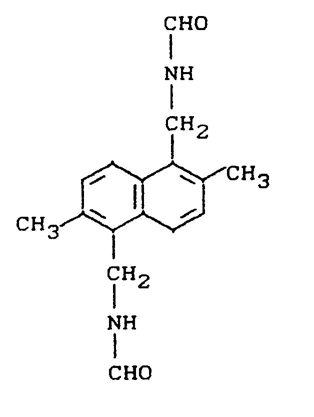

Zu einer Mischung von 35,1 g 2,6-Dimethylnaphthalin, 240 ml Eisessig und 60 ml konzentrierter Schwefelsäure wird bei 40°C in 1,5 Stunden eine N-Methylolformamid-Lösung, hergestellt aus 30 ml Formamid, 17,3 g Paraformaldehyd und 0,3 ml 40 %iger Natronlauge, zugetropft. Nach 7 Stunden bei 40 °C rührt man kalt über Nacht nach, kühlt dann auf ca. 5°C ab, saugt den Niederschlag ab und wäscht ihn mit 50 ml Eisessig und Wasser nach. Das Rohprodukt wird in 80 ml Methanol verrührt, abgesaugt und zweimal mit Methanol gewaschen. Ausbeute 11,7 g.

### Stufe 2

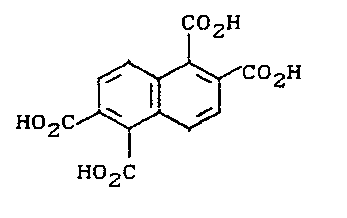

6,75 g des obigen Produktes werden mit 56 g Natriumbichromatdihydrat und 112 ml Wasser 30 Stunden auf 200 bis 210°C erhitzt. Anschließend filtriert man vom Chromoxid ab und säuert das Filtrat mit ca. 80 ml 15 %iger Schwefelsäure an. Der Niederschlag wird abgesaugt und gut mit Wasser gewaschen. Ausbeute 3,3 g.

### Beispiel 22

### Stufe 1

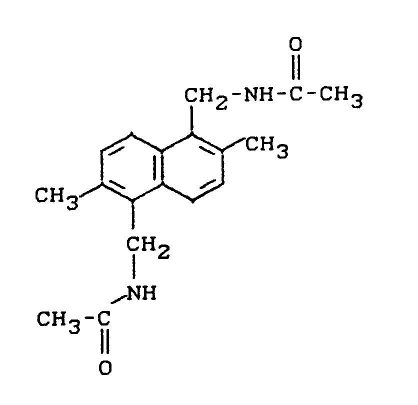

Eine Mischung aus 11,7 g 2,6-Dimethylnaphthalin, 80 ml Eisessig, 20 ml konzentrierte Schwefelsäure und 17,4 g N-Methylolacetamid wird 6 Stunden bei 40°C gehalten. Das ausgefallene Produkt wird abgesaugt, mit Eisessig und Wasser gewaschen. Ausbeute 8,4 g.

### Stufe 2

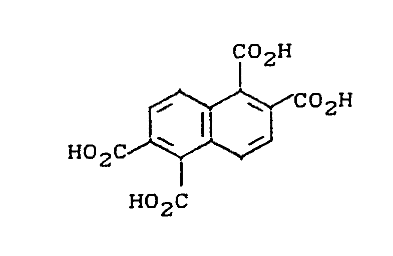

Die zu Beispiel 21, Stufe 2 analoge Oxidation von 7,45 g des Produktes der Stufe 1 ergibt 3,6 g Naphthalin-1,2,5,6-tetracarbonsäure.

### Beispiel 23

### Stufe 1

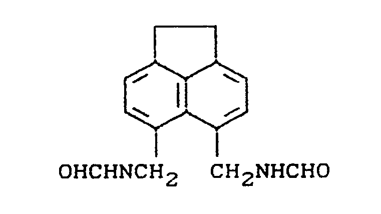

Zu einer Mischung aus 11,5 g Acenaphthen, 80 ml Eisessig und 20 ml 100%ige Schwefelsäure wird während 15 Minuten bei 40°C eine Methylolformamid-Lösung (hergestellt aus 10 ml Formamid, 5,75 g Paraformaldehyd und 0,1 ml 40%ier Natronlauge) zugetropft. Nach 6 Stunden bei 40 bis 45°C wird die Mischung auf 500 ml Eis ausgetragen, der Niederschlag nach dem Stehen über Nacht abgetrennt und mit Wasser gewaschen. Ausbeute 13,4 g.

### Stufe 2

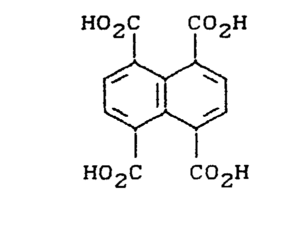

Oxidation von 13,0 g des obigen Produktes und Aufarbeitung analog zu Beispiel 11, Stufe 2 liefern 4,0 g 1, 4, 5, 8-Naphthalintetracarbonsäure.

## Patentansprüche

1. Verfahren zur Herstellung von Naphthalinpolycarbonsäuren, dadurch gekennzeichnet, daß man Alkylnaphthaline mit einem oder mehreren Säureamiden der Formel (2) 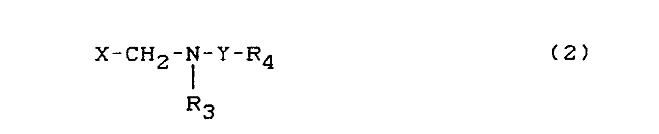
ein- oder mehrfach bei einer Temperatur zwischen 0 und 200°C amidomethyliert und die dabei entstandenen Zwischenprodukte oder deren Hydrolysate bzw. deren Salze zu Naphthalinpolycarbonsäuren oder deren Salzen oxidiert, wobei die Substituenten in Formel (2) folgende Bedeutung haben:
X = OH, Alkoxy, Halogen, Dialkylamino, Trialkylammonium oder cyclisches Amin, das durch weitere Heteroatome unterbrochen sein kann oder 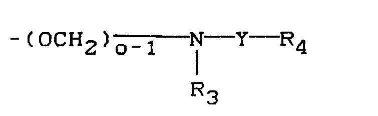
Y = CO, CS, C=N-R₅, S02,
R₃ = Wasserstoff, gegebenenfalls substituiertes C₁-C₁₀-Alkyl, -Alkenyl, -Alkinyl oder Phenyl,
R₄ = Wasserstoff, OH, gegebenenfalls substituiertes C₁-C₁₀-Alkyl, -Alkenyl, -Alkinyl, Phenyl, Amino, C₁-C₄-Alkoxy, Alkoxycarbonyl oder Aminocarbonyl,
oder
R₃ und R₄ gemeinsam den Rest eines gegebenenfalls von weiteren Heteroatomen unterbrochenen aliphatischen oder aromatischen Ring bilden,
R₅ = Wasserstoff, gegebenenfalls substituiertes C₁-C₆-Alkyl oder gegebenenfalls substituiertes Phenyl,

2. Verfahren gemäß Anspruch 1, dadurch gekennzeichnet, daß man in der ersten Stufe Alkylnaphthaline der Formel (1) 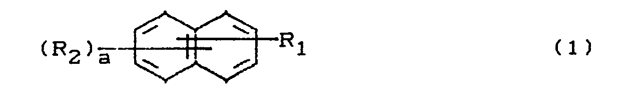
mit einem oder mehreren Säureamiden der Formel (2) in einer Einhorn-Reaktion zu Verbindungen der Formel (3a) 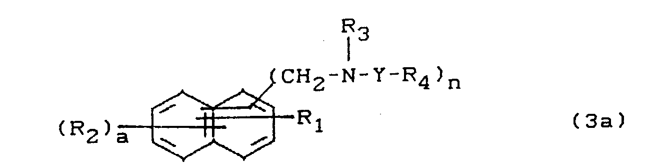
umsetzt und diese oder deren Hydrolysate, die den Aminen der Formel (3b) bzw. deren Salze entsprechen 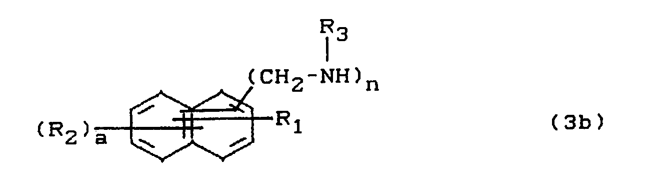
in einer zweiten Stufe zu Naphthalin-polycarbonsäuren oder deren Salze oxidiert, worin
R₁ und R₂ = unabhängig voneinander Alkyl bedeuten oder zusammen eine C₂-C₆-Alkyleneinheit bilden,
a = 0 oder 1 und
n = 1, 2 oder 3 bedeutet.

3. Verfahren gemäß Anspruch 1, dadurch gekennzeichnet, daß Säureamide der Formel (2) eingesetzt werden, worin Y = CO und X = OH bedeuten.

4. Verfahren gemäß Anspruch 2, worin bedeuten:
Y = CO,
Rᵢ, R₂ = unabhängig voneinander Methyl, Ethyl, Propyl, Butyl oder zusammen eine Ethylen-oder Propylen-Einheit,
X = OH, C₁-C₄-Alkoxy, Cl, Br, Di-(C₁-C₆-alkyl)-amino 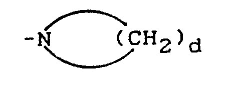
worin d = 4 bis 6,
Morpholinyl, N-C₁-C₄-Alkyl-piperazinyl oder Tri-(C₁-C₆-alkyl)-ammonium mit den Gegenionen Chlorid, Bromid, lodid, Tosylat, C₁-C₆-Alkylsulfat oder Sulfat oder 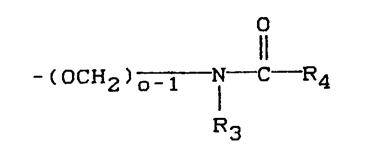
R₃ = Wasserstoff, gegebenenfalls durch Cl, OH, C₁-C₄-Alkoxy, Di-(C₁-C₄-alkyl)-amino, CN, C₁-C₄-Alkoxycarbonyl oder Aryl substituiertes C₁-C₁₀-Alkyl, -Alkenyl oder -Alkinyl oder gegebenenfalls durch CI oder C₁-C₄-Alkyl substituiertes Phenyl,
R₄ = Wasserstoff, gegebenenfalls durch F, Cl, Br, CN und/oder Aryl einfach oder mehrfach substituiertes C₁-C₁₀-Alkyl, -Alkenyl oder -Alkinyl, gegebenenfalls durch Cl, OH, Amino, Acylamino oder Methyl substituiertes Phenyl, Amino, Mono- oder Di-(C₁-C₄-alkyl)-amino, gegebenenfalls durch Phenyl substituiertes C₁-C₄-Alkoxy, C₁-C₄-Alkoxycarbonyl, Aminocarbonyl oder OH
oder R₃ und R₄ gemeinsam die folgenden Formeln bilden: 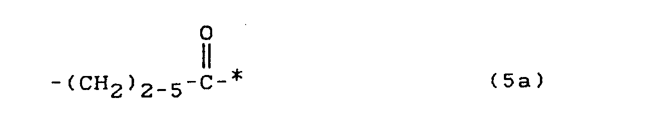 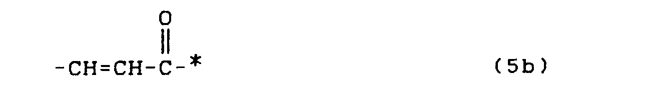 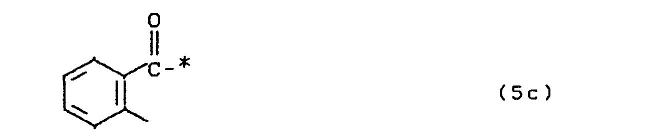 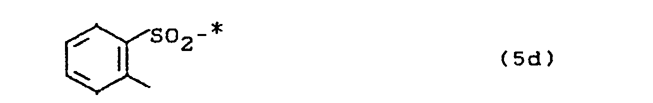 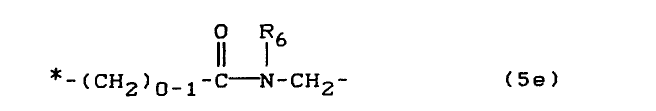 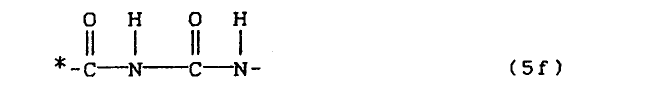 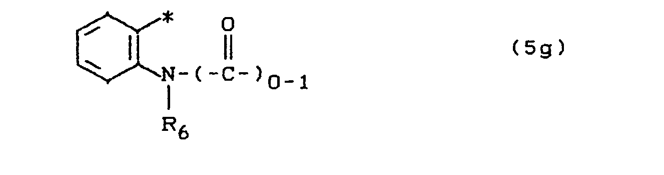
oder
-(CH₂)₃₋₅- (5h),
worin R₆ = Wasserstoff, C₁-C₄-Alkyl, Acyl oder Aryl, mit der Vorgabe, daß die mit einem ^{*} gekennzeichneten Stellen in den Formeln (5a) bis (5g) immer mit dem N-Atom der Formel (2) verknüpft sind.

5. Verfahren gemäß Anspruch 2, dadurch gekennzeichnet, daß Alkylnaphthaline der Formel (1) aus der folgenden Gruppe eingesetzt werden:

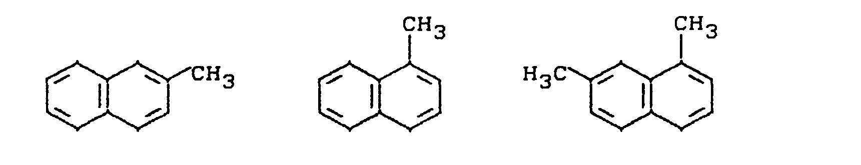

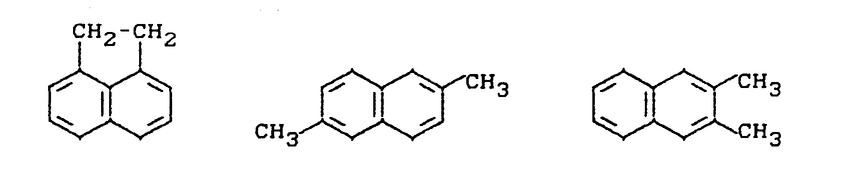

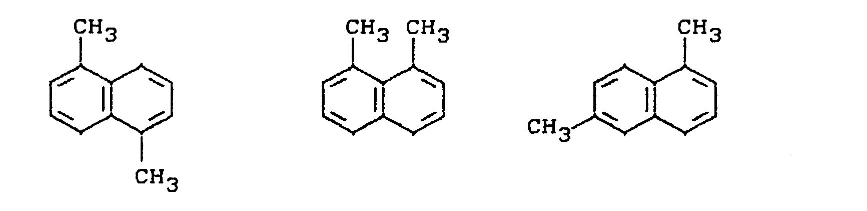

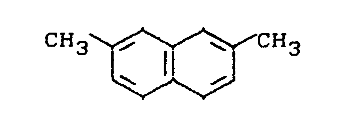

6. Verfahren gemäß Anspruch 2, dadurch gekennzeichnet, daß polyfunktionelle Säureamide der Formel (2) eingesetzt werden, worin
R₃ und/oder R₄ unabhängig voneinander weitere zu einer Einhorn-Reaktion fähige Struktureinheiten der Formel 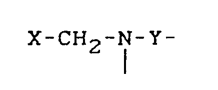
insbesondere 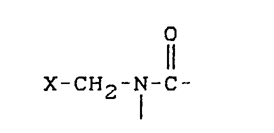
enthalten oder zusammen solche Struktureinheiten enthaltende Reste bilden, worin X und Y die in Anspruch 1 genannten Bedeutungen besitzen.

7. Verfahren gemäß Anspruch 1, dadurch gekennzeichnet, daß als Lösungsmittel für die Einhorn-Reaktion Ameisensäure, Essigsäure, Propionsäure, deren C1-C4-Alkylester oder Acetanhydrid und als Katalysator für die Einhorn-Reaktion Schwefelsäure, Oleum, Phosphorsäure oder Polyphosphorsäure eingesetzt wird.

8. Verfahren gemäß Anspruch 1, dadurch gekennzeichnet, daß als Oxidationsmittel Alkalichromate und/oder -bichromate, insbesondere Natriumbichromat eingesetzt werden und als Lösungsmittel für die Oxidation Wasser, aliphatische Carbonsäuren, wäßrige Mineralsäure, Alkali-/Erdalkalilaugen, Halogenaromaten oder Halogenaliphaten eingesetzt werden.

9. Verbindungen der Formel (3a) 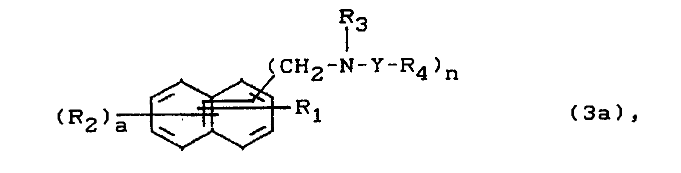
worin
Ri, R₂, R₃, R₄, a, n und Y die in Anspruch 2 genannten Bedeutungen haben mit Ausnahme der Verbindungen: 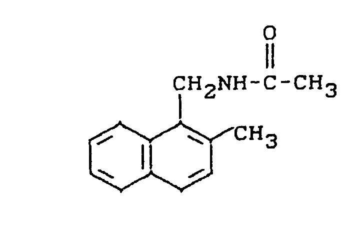 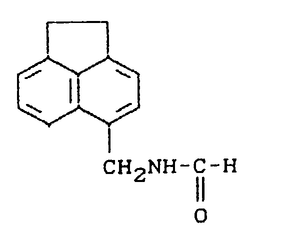 und

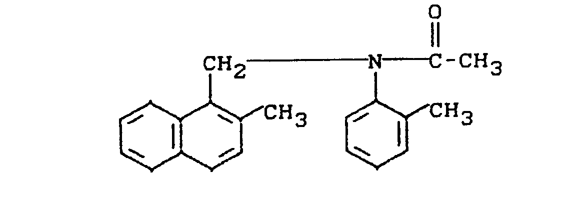

10. Verbindungen der Formel (3b) oder deren Salze, 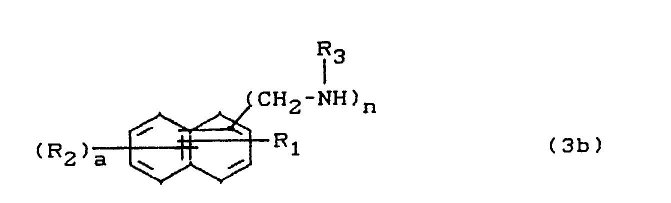
worin
Ri, R₂, R₃, R₄, a und n die in Anspruch 2 genannten Bedeutungen haben mit Ausnahme der Verbindungen: 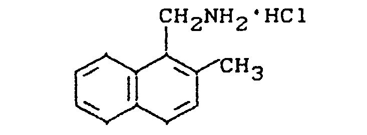 und
